# EUROPEAN PATENT APPLICATION

(11) **EP 3 016 061 A1**
(43) Date of publication of application: **04.05.2016**
(21) Application number: 15003141.7
(22) Date of filing: 03.11.2015
(51) Int. Cl.: G06Q 50/22

(54) **DISCOVERY OF INCENTIVE EFFECTIVENESS**

(30) Priority: 03.11.2014 US 201414531863
(71) Applicant: Google Inc., Mountain View, CA 94043 (US)
(72) Inventor: Chou, Katherine, Mountain View, CA 94043 (US); Jindal, Deepak, Mountain View, CA 94043 (US); Tang, Diane, Mountain View, CA 94043 (US); Syed, Zeeshan, Mountain View, CA 94043 (US); Davis, Geoffrey Mark, Mountain View, CA 94043 (US); Roat, Christopher, Mountain View, CA 94043 (US); Stanis, Thomas Randolph, Mountain View, CA 94043 (US); Moisa, Dan, Mountain View, CA 94043 (US)
(74) Representative: Johnson, Richard Alan

(57) **Abstract**

A method comprising:
compiling, for each of a plurality of individuals, health-related data in a plurality of categories;
determining that a given individual in the plurality of individuals has a particular type of health-related data in a particular set of one or more categories;
based on the determination that the given individual has the particular type of health-related data in the particular set of one or more categories, transmitting from a server device to a client device associated with the given individual, over a communication network, a first instruction configured to cause the client device to present a first incentive designed to cause a change in the given individual's health-related data, wherein the first incentive makes use of a first type of motivational foundation; and
after transmitting the first instruction, determining whether the first incentive was effective or ineffective.

## Description

### BACKGROUND

Unless otherwise indicated herein, the materials described in this section are not prior art to the claims in this application and are not admitted to be prior art by inclusion in this section.

Computing systems such as personal computers, laptop computers, tablet computers, cellular phones, and countless types of Internet-capable devices are prevalent in numerous aspects of modern life. Over time, the manner in which these devices are providing information to users is becoming more intelligent, more efficient, more intuitive, and/or less obtrusive. Additionally, computing systems may be used to collect, store, and process various types of data relating to a user in order to provide helpful recommendations, visualizations, or other communications regarding the data.

### SUMMARY

Some embodiments of the present disclosure provide a method that includes compiling, for each of a plurality of individuals, health-related data in a plurality of categories, determining that a given individual has a particular type of health-related data in a particular set of one or more categories, and based on the determination that the given individual has the particular type of health-related data in the particular set of one or more categories, transmitting from a server device to a client device associated with the given individual, over a communication network, a first instruction configured to cause the client device to present a first incentive designed to cause a change in the given individual's health-related data. The first incentive may make use of a first type of motivational foundation. The method may also include determining whether the first incentive was effective or ineffective.

Additionally, some embodiments of the present disclosure provide a system than includes one or more processors, a communication interface, and computer-readable storage media having stored thereon instructions that, when executed by the one or more processors, cause the system to engage in operations. In some embodiments, the operations include compiling, for each of a plurality of individuals, health-related data in a plurality of categories, determining that a given individual in the plurality of individuals has a particular type of health-related data in a particular set of one or more categories, and based on the determination that the given individual has the first type of health-related data in the particular set of one or more categories, transmitting from the system via the communication interface to a client device associated with the given individual a first instruction configured to cause the client device to present a first incentive designed to cause a change in the given individual's health-related data. The first incentive may use of a first type of motivational foundation. The operations may also include determining whether the first incentive was effective or ineffective.

These as well as other aspects, advantages, and alternatives, will become apparent to those of ordinary skill in the art by reading the following detailed description, with reference where appropriate to the accompanying drawings.

### BRIEF DESCRIPTION OF THE DRAWINGS

Figure 1 is a block diagram of an example system that includes a server system, a plurality of data sources, and a plurality of client devices in communication across a network, in accordance with an example embodiment.
Figure 2 is a functional block diagram of an example server system, in accordance with an example embodiment.
Figure 3 is an example chart depicting example motivational foundations, in accordance with an example embodiment.
Figure 4 is a flowchart of an example method, in accordance with an example embodiment.

### DETAILED DESCRIPTION

In the following detailed description, reference is made to the accompanying figures, which form a part hereof. In the figures, similar symbols typically identify similar components, unless context dictates otherwise. The illustrative embodiments described in the detailed description, figures, and claims are not meant to be limiting. Other embodiments may be utilized, and other changes may be made, without departing from the scope of the subject matter presented herein. It will be readily understood that the aspects of the present disclosure, as generally described herein, and illustrated in the figures, can be arranged, substituted, combined, separated, and designed in a wide variety of different configurations, all of which are explicitly contemplated herein.

### I. Overview

Example embodiments may relate to methods and systems for determining the types of incentives that are effective in motivating an individual to change his or her health state and thereafter providing incentives in accordance with the determination.

In order to encourage an individual to engage in actions or inactions that lead to a desired change in the individual's health-related data, a health system may be configured to present one or more incentives to the individual. In some examples, an incentive may take the form of a message, such as a text message or an email message, displayed on a graphical user interface of a wearable (or non-wearable) computing device.

Different individuals may be motivated to engage in one or more actions (such as exercising) or inactions (such as refraining from smoking) based on different types of incentives. Thus, to facilitate presenting an individual with an incentive that is effective in motivating the individual to engage in a health-related action or inaction, the system may engage in an incentive-discovery process in order to develop an incentive profile for the individual. The system may then present incentives to the individual in accordance with the individual's incentive profile.

In accordance with one example of the incentive-discovery process, the system presents to a given individual one or more incentives classified as a particular type (*e.g.,* extrinsically motivational and positive reinforcement) and directed at a particular type of health-related data (*e.g.,* the individual's body-mass index (BMI)). The system determines how the given individual responds to the particular type of incentive. When the given individual engages in an action or inaction directed at the particular type of health-related data, the system may consider the particular type of incentive to be effective. On the other hand, when the given individual fails to engage in an action or inaction directed at the particular type of health-related data, the system may consider the particular type of incentive to be ineffective.

As an alternative, or in addition to, determining the response by the individual, the system may analyze the individual's health-related data (*e.g.,* the individual's BMI) to determine whether the particular type of health-related data underwent a desired change. If the particular type of health-related data underwent the desired change, then the system may consider common incentives (and the types thereof) presented to the individual during the time the health-related data underwent the desired change to be effective. On the other hand, if the particular type of health-related data did not undergo the desired change, then the system may consider common incentives (and the types thereof) presented to the individual during the time the health-related data did not undergo the desired change to be ineffective.

As a result of determining that certain types of incentives are effective for an individual and other types of incentives are not effective, the system may thereafter present to the individual the effective incentives more often than the system presents to the individual the ineffective incentives. Additionally, as a result of engaging in the incentive-discovery process for a population of individuals, the system may identify patterns of effective and ineffective incentive types among individuals with certain sets of common demographic data. Thus, for a given individual that shares this common set of demographic data, the system may present to the given individual the effective incentives more often than the system presents to the given individual the ineffective incentives, even if the system has not (yet) engaged in the incentive-discovery process for the given individual.

### II. Example Health System

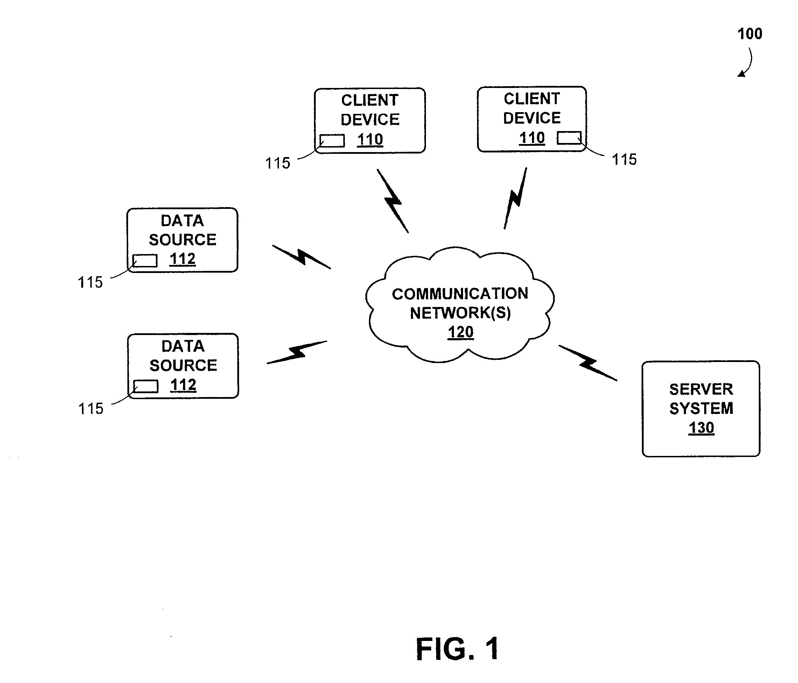

Figure 1 is a simplified schematic of an example health system 100 that includes a server system 130, one or more client devices 110, and one or more data sources 112, all of which are communicatively coupled across one or more communication networks 120. The one or more client devices 110 and the one or more data sources may be configured to transmit health-related data via respective communication interfaces 115 over the one or more communication networks 120 to the server system 130. Server system 130 may be configured to, among other things, correlate the health-related data received over networks 120 from a population of individuals, identify patterns in the health-related data based on the correlation, and provide health-related alerts, recommendations, and/or incentives to individuals via client devices 110.

The server system 130 may include any type of remote computing device or remote cloud computing network. The server system 130 may be configured to compile from the client devices 110 and data sources 112 health-related data associated with many different individuals. This health-related data may be assorted into various categories, which, by way of example, may include demographic data, environmental data, behavioral data, clinical data, and biomarker data, among other examples. Demographic data may include data related to an individual's age, height, weight, gender, ethnicity, occupation, residence city, state, or region, among other examples. Environmental data may include data related to the particular environment in which an individual is located, including for instance, air quality measurements, air pressure, relative humidity, temperature, elevation, weather patterns, average amount of sun exposure per day, among other examples. Behavioral data may include data related to diet, sleep pattern, and/or the type, duration, and intensity of any physical activity in which an individual engages, among other examples. Clinical data may include data generated by or determined with the aid of a clinician, including, for instance, the type and dosage of prescription drug usage, and/or diagnosis of medical condition(s). The biomarker data may include data determined with the aid of a clinician as well. But biomarker data may relate more specifically to physiological parameter measurements that tend to be indicators of the presence or absence of disease state(s), including for instance, blood pressure, pulse rate, respiration rate, body temperature, and/or measurements related to cholesterol, glucose, white blood cell, red blood cell, among other examples. In addition to these data categories, the server system 130 may compile from the client devices and data sources health-related data in other categories as well.

Client devices 110 may include any computing device associated with an individual and capable of collecting, transmitting, and/or receiving health-related data or alerts, recommendations, or incentives regarding health-related data. Example client devices may include mobile telephones, personal or tablet computers, and/or wearable computing devices, among others. In some examples, a client device may measure or otherwise receive health-related data directly from an individual. For instance, the client devices may include a personal computer or mobile telephone, on which an individual may establish a user account and may from time to time input various health-related data, such as demographic data, environmental data, and/or behavioral data.

In another example, the client devices may include a wearable device that is capable of being worn at, on, or in proximity to an external body surface, such as a wrist, ankle, waist, chest, head, or other body part, and is configured to measure certain physiological parameters of a person wearing the device. For instance, some wearable devices may be configured with various electronic and mechanical components that facilitate the measurement of such parameters as blood pressure, pulse rate, respiration rate, skin temperature, galvanic skin response (GSR), sleep patterns, as well as the type, duration, and intensity of physical activity engaged in by the wearer of the wearable device. For instance, a wearable device may collect data indicating that the wearer engaged in a running activity for 30 minutes on a particular date and at a particular time. The data may also indicate location coordinates of a course taken by the wearer during the physical activity, as well as perhaps indications of health-related physiological parameter measurements (*e.g.,* blood pressure, pulse rate, respiration rate, skin temperature, GSR, etc.) of the wearer taken by the wearable device during the physical activity. Other wearable devices and other client devices can collect and transmit to the server system 130 other types of health-related data as well.

Data sources 112 may include any other device that is typically not directly associated with an individual but still capable of transmitting or receiving health-related data pertaining to an individual. For instance, data sources 112 may include computing devices or data storage associated with an individual's heath professional, which may contain recent medical test results for an individual as well as individual's overall medical history. The data sources may include computing devices or data storage containing environmental or geographical data relevant to an individual, such as the National Weather Service or other similar organizations. The data sources may also include computing devices or data storage containing population-wide health data that may be relevant to certain individuals, such as Centers for Disease Control (CDC). Other data sources are possible as well.

Individuals associated with client devices 110 and data sources 112 may be provided with an opportunity to control whether or how the respective device collects health-related information about the wearer, and/or to control how such information may be used. Thus, an individual may have control over how information is collected about him or her and used by the server system 130. For example, an individual may elect that data, such as health state and physiological parameters, collected from a client device may only be used for generating an individual baselines and recommendations in response to collection and comparison of his or her own data and may not be used in generating a population baseline or for use in population correlation studies.

Further, some embodiments of the system 100 may include privacy controls which may be automatically implemented or controlled by individuals associated with the client devices 110 and data sources 112. For example, where an individual's health-related data are uploaded to the server system 130, the data may be treated in one or more ways before it is stored or used, so that personally identifiable information is removed. For example, an individual's identity may be treated so that no personally identifiable information can be determined for the individual, or an individual's geographic location may be generalized where location information is obtained (such as to a city, ZIP code, or state level), so that a particular location of an individual cannot be determined.

As further depicted in Figure 1, data sources 112 and client devices 110 may include respective communication interfaces 115 that comprise a wireless transceiver for sending and receiving communications to and from the server system 130. In other cases, the communication interface 115 may include any means for the transfer of data, including both wired and wireless communications. For example, the communication interfaces 115 may include a universal serial bus (USB) interface or a secure digital (SD) card interface.

Client devices 110 and/or data sources 112 may also include respective user interfaces via which an individual associated with the device or data source may receive one or more alerts, recommendations, or incentives generated by the server system 130 or other remote computing device, or from a processor within the client device itself. The alerts, recommendations, or incentives could be any indication that can be noticed by the associated individual. For example, an alert, recommendation, or incentive may include a visual component (*e.g.,* textual or graphical information on a display), an auditory component *(e.g.,* an alarm sound), and/or tactile component (*e.g.,* a vibration). Further, a respective user interface may include, by way of example, a display on which a visual indication of the alert, recommendation, or incentive may be displayed.

Communication networks 120 may generally be any network or combination of networks that facilitate the transmission of health-related data between the client devices 110, data sources 112, and server 130. Such networks may include any of: a plain old telephone service (POTS) network, a cellular network, a fiber network, and a data network. Further, communication networks 120 may include one or more intermediaries, including, for example wherein the client devices 110 and the data sources 112 transmit data to an additional computing device, such as a mobile phone or other personal computing device, which in turn transmits the data to the server system 130.

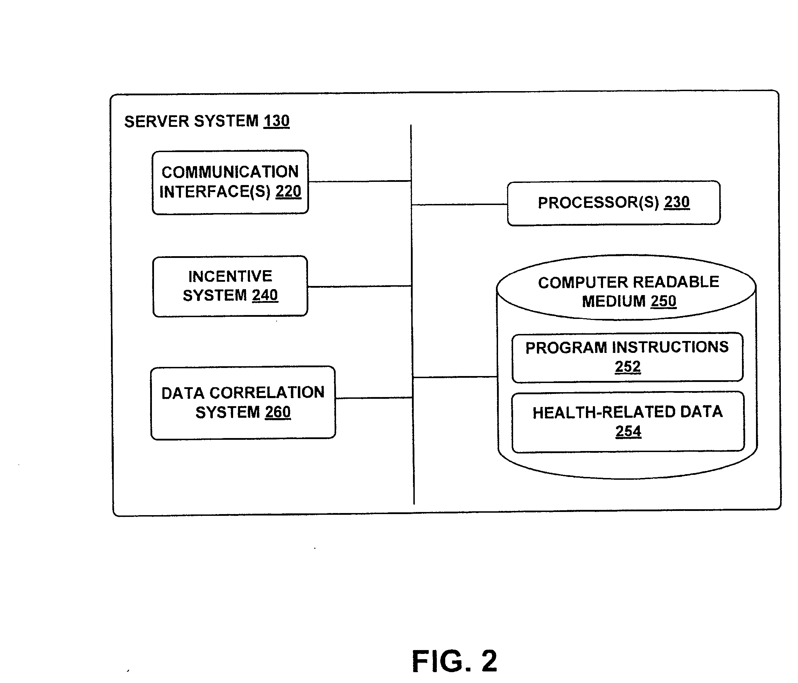

Figure 2 a simplified block diagram depicting example components of server system 130, according to an example embodiment. In particular, Figure 2 shows an example of server system 130 having one or more communication interfaces 220, one or more processors 230, an incentive system 240, a computer-readable medium 250, and a data correlation system 260, all of which are communicatively coupled via a system bus or other mechanism. Other examples of a server system may include more or fewer components.

Generally, communication interface(s) 220 may be any wired (e.g., Ethernet) or wireless (e.g., using Wi-Fi or another wireless communication protocol) interface capable of communicating with another entity (*e.g*., client devices 110 and data sources 112). Communication interface(s) 220 may be operated by the one or more processors 230 via the execution of program instructions 250.

Processor(s) 230 may include a general-purpose processor or a special purpose processor (*e.g.,* digital signal processors, application specific integrated circuits, etc.). The one or more processors 230 can be configured to execute computer-readable program instructions 252 that are stored in a computer readable medium 250 and are executable to provide the functionality of a server system 130 as described herein. The computer readable medium 250 may further contain the health-related data 254 compiled from the client devices 110 and data sources 112.

The computer readable medium 250 may include or take the form of one or more non-transitory, computer-readable storage media that can be read or accessed by at least one processor 230. The one or more computer-readable storage media can include volatile and/or non-volatile storage components, such as optical, magnetic, organic or other memory or disc storage, which can be integrated in whole or in part with at least one of the one or more processors 230. In some embodiments, the computer readable medium 250 can be implemented using a single physical device (*e.g.,* one optical, magnetic, organic or other memory or disc storage unit), while in other embodiments, the computer readable medium 250 can be implemented using two or more physical devices.

The program instructions 252 stored on the computer readable medium 250 may include instructions to perform or facilitate some or all of the device functionality described herein. For instance, program instructions 252 may include instructions to operate the communication interface(s) 220 to poll the client devices and/or data sources in order receive health-related data for individuals.

Server system 130 may include additional systems, such as an incentive system 240 and a data correlation system 260. In some embodiments, these additional systems may be separate computing systems that make up part of the server system 130. As such, the additional systems may include their own processors (not shown) and computer readable storage media (not shown) with program instructions executable to cause the server system 130 (and more particularly, the other individual components of server system 130) to carry out functions. In other embodiments, the additional systems may be individual program modules of program instructions 252 stored in computer readable medium 250 and executable by the processor(s) 230 to carry out additional functionality. Other examples are possible as well.

The data correlation system 260 may be configured to analyze the health-related data 254 compiled for a population of individuals and carry out certain functions based on this analysis. In some examples, the data correlation system 260 may identify patterns among the health-related data, identify changes in health-related data that are indicative of various health-states. In response to identifying a particular pattern or coming to a particular conclusion regarding the health-related data of a particular individual, the data correlation system may cause the server system 130 to transmit an alert, recommendation, or incentive to a client device associated with that particular individual.

In some examples, data correlation system 260 may be used to make determinations regarding the efficacy of a drug or other treatment based on the health-related data, which may include information regarding the drugs or other treatments received by an individual, physiological parameter data for the individual, and/or an indicated health state of the individual. From this information, the data correlation system 260 may be configured to derive an indication of the effectiveness of the drug or treatment. For example, if an individual's health-related data indicates that the individual is using a drug intended to treat nausea and other health-related data for the individual indicates that he or she has not experienced nausea for some time after beginning a course of treatment with the drug, the data correlation system 260 may be configured to derive an indication that the drug is effective for that individual.

In another example, health-related data for an individual may indicate the individual's blood glucose level over a period of time. If that individual is prescribed a drug intended to treat diabetes, but the data correlation system 260 determines that the individual's blood glucose has been increasing over a certain number of measurement periods, the data correlation system 260 may be configured to derive an indication that the drug is not effective for its intended purpose for that individual.

In some examples, data correlation system 260 may analyze an individual's health-related data to determine that a particular medical condition is indicated. Responsively, the data correlation system 260 may cause the server 130 to generate and transmit an alert to an associated client device 110. As noted above, the alert may include a visual component, such as textual or graphical information displayed on a display, an auditory component (*e.g.,* an alarm sound), and/or tactile component (*e.g.,* a vibration). The textual information may include one or more recommendations, such as a recommendation that the individual of the device contact a medical professional, seek immediate medical attention, or administer a medication.

As also depicted in Figure 2, server system 130 may include an incentive system 240. Incentive system 240 may be configured to generate an incentive designed to motivate or encourage an individual to engage in one or more behaviors in order to change part of the individual's health-related data. For instance, an incentive may be designed to encourage an individual to exercise more, take a particular drug prescribed for the individual, stop smoking, use sunscreen, or engage in any other action or inaction to change part of the individual's health related data. An incentive may generally take any form, including a message, alert, recommendation, or other communication presented at a client device associated with an individual. In some examples, an incentive may include a visual component, such as textual or graphical information displayed on a display, an auditory component (*e.g.,* an alarm sound), and/or a tactile component (*e.g.,* a vibration).

In practice, different individuals may be motivated in different ways. For example, some individuals may be more motivated by positive reinforcement, whereas other individuals may be more motivated by negative reinforcement. Likewise, some individuals may be more motivated by extrinsic factors, whereas other individuals may be more motivated by intrinsic factors. Still others individuals may be more motivated in other ways as well. Further, the type of motivation most effective for a given individual may yet be different depending on the type of behavior encouraged. For instance, a given individual may be more motivated by negative reinforcement to stop smoking, whereas the same individual may be more motivated by positive reinforcement to start (or continue) exercising.

To this end, the incentive system 240 may develop an incentive profile for a given individual and construct or select incentives for that individual that make use of a particular type of motivational foundation (*e.g.,* positive reinforcement, negative reinforcement, extrinsic motivations, intrinsic motivations, and/or another type of motivational foundation) based on the incentive profile. For instance, an incentive that makes use of a positive reinforcement may be arranged with a relatively positive tone, or offer or explain how a certain behavior will lead to a positive consequence. On the other hand, an incentive that makes use of a negative reinforcement may be arranged with a relatively negative tone, or offer or explain how a certain behavior will lead to a negative consequence. Further, an incentive that makes use of an intrinsic motivation may be arranged to present the individual's own health-related data in one form or another. On the other hand, an incentive that makes use of an extrinsic motivation may be arranged to present other individuals' health-related data in one form or another, perhaps in comparison to the individual's own health-related data.

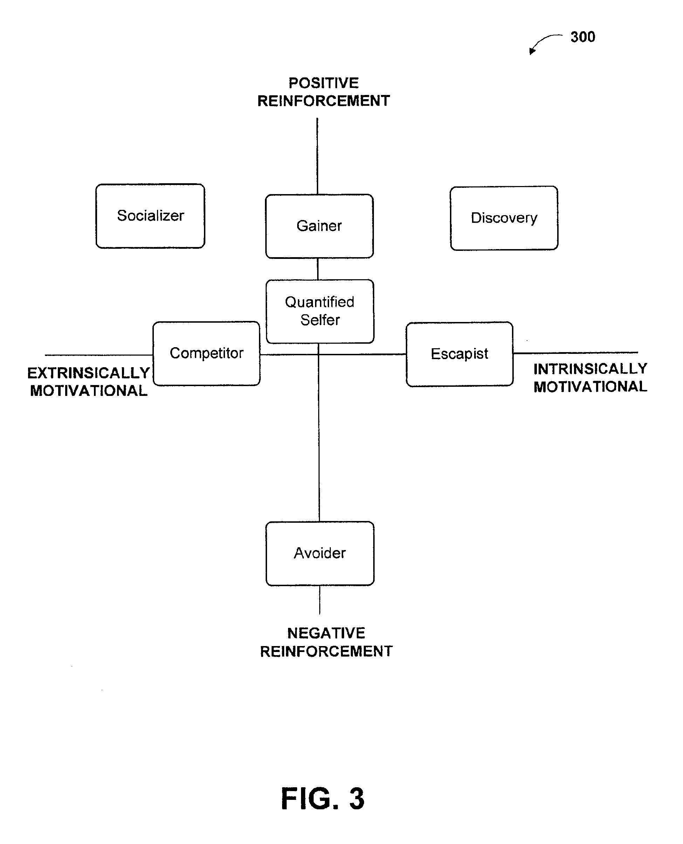

In order to more fully illustrate how some motivational foundations are used with different incentive profiles, Figure 3 depicts a chart 300 of several types of example incentive profiles. As depicted, the example incentive profiles include Socializer, Competitor, Gainer, Quantified-Selfer, Avoider, Escapist, and Discovery, although other profiles are possible as well. Each example incentive profile is depicted somewhere on the chart 300 depending on the type of motivational foundation or foundations that tend be most effective for that type of incentive profile.

For example, for an individual classified as a Socializer, the incentive system 240 may utilize an incentive that makes use of positive reinforcement and an extrinsic motivation, such as a complimentary message from one or more of the individual's friends. For an individual classified as a Competitor, the incentive system 240 may utilize an incentive that makes use of an extrinsic motivation that may compare the individual's health-related data to other individuals' health-related data, such as a message that reads, "90% of other 32 year old women in your city can run a mile in under 10 minutes." For an individual classified as a Gainer, the incentive system 240 may utilize an incentive that makes use of positive reinforcement and compares the individual's contemporary health-related data to the individual's historical health-related data, such as with a message that reads, "You have run over 15 miles this week, bringing your year-to-date total to 75 miles," or "You have decreased your average mile time from 10 minutes to 9 minutes." For an individual classified as a Quantified Selfer, the incentive system 240 may utilize an incentive that makes use of positive reinforcement and presents the individual's health-related data in various ways, such as with a message that reads, "You blood pressure currently is 120 / 80 and have a resting pulse rate of 62 bpm." For an individual classified as an avoider, the incentive system 240 may utilize an incentive that makes use of negative reinforcement and presents example negative consequences for engaging in certain behaviors. For an individual classified as an Escapist, the incentive system 240 may utilize an incentive that makes use of an intrinsic motivation that may present the individual's health related data in ways that represent alternative realities, such as if the individual existed in a game world or a historical setting. And for an individual classified as Discovery, the incentive system 240 may utilize an incentive that makes use of positive reinforcement and an intrinsic motivation that encourages the individual to participate in something new, such as a new exercise route or new software testing. It will be appreciated that the statistics and values regarding the health-related data presented above are merely examples; in other examples, other statistics and other values are possible. Additionally, other incentive profiles may exist as well that make use of other types of motivations.

### III. Example Incentive Discovery

In practice, incentive system 240 may generate incentives designed to motivate or encourage an individual to engage in one or more behaviors in order to change part of the individual's health-related data. In one example, these incentives may be generated in response to certain goals indicated by the individual (or someone associated with the individual, such as the individual's healthcare professional). For example, an individual's health-related data may indicate that the individual has a goal to lose 15 pounds within one year. Responsively, the incentive system 240 may generate incentives that are designed to encourage the individual to exercise more, change the individual's diet, or engage in any other behavior to meet this goal. In other examples, incentives may not be generated in response to any particularly indicated goal, but rather, the incentive system 240 may generate incentives designed to generally promote health.

Incentives may be pre-programmed and stored in data storage in computer-readable medium 250. Additionally, incentives may be tagged or classified depending on the type or types of motivational foundation(s) of which the incentive makes use. Depending on the type of incentive desired to be used, incentive system 240 may refer to data correlation system 250 to determine statistics relating to individuals' health-related data in order to present the statistics in the incentive. For instance, if incentive system 240 is generating an incentive for a particular individual, the incentive system 240 may refer to data correlation system 250 and health-related data 254 to determine where some of the particular individual's health-related data ranks among health-related data of other individuals with similar ages, with similar residencies, similar careers, or any other similarity in health-related data.

As noted above, incentive system 240 may construct or select incentives for a given individual that makes use of a particular type of motivational foundation based on the incentive profile of the given individual. Thus, health-related data 254 may contain incentive-profile data that indicates an incentive profile for the given individual. Incentive-profile data may include data that specifies a particular one of the example incentive profiles discussed above with respect to Figure 3; however, the incentive-profile data may additionally or alternatively specify the type or types of motivational foundations considered effective in motivating the given individual to engage in one or more behaviors to change the individual's health-related data.

Initially, incentive-profile data for a given individual may be generated based on the individual's health-related data itself. For instance, it may be known that, on average, individuals aged 30-50 with yearly incomes of $50,000 - $100,000 are most effectively motivated with positive reinforcement and intrinsic motivational foundations. Thus, incentive-profile data for these individuals may contain indications that positive reinforcement and intrinsically motivational foundations are effective. When incentive system 240 generates or selects an incentive for a given one of these individuals, the incentive system may refer to the incentive-profile data, determine that positive reinforcement and intrinsic motivations are most effective, and select or construct an incentive accordingly. Other examples of effective motivational foundations are possible for individuals having other types of health-related data as well.

Even though individuals sharing similar demographic data (or other health-related data) may, on average, tend to be motivated by the same motivational foundations, it may often the case that many individuals are not similarly motivated. Therefore, the incentive system 240 and server system 130 may engage in an incentive discovery process for an individual in an effort to provide more effective incentives to the individual. An incentive discovery process may help the incentive system 240 to determine which type or types of motivational foundations are effective for the given individual. The incentive system may modify the individual's incentive-profile data to indicate which type or types of incentives are effective, and the incentive system may thereafter present the individual with incentive in accordance with the individual's new incentive profile. Additionally or alternatively, after conducting several iterations of the incentive discovery process for several individuals, the incentive system and data correlation system may identify new patterns of effective motivational foundations for individuals sharing similar health-related data. The incentive system may responsively modify incentive-profile data of other individuals sharing the similar health-related data in accordance with the determined patterns. Other benefits and other actions are possible as well.

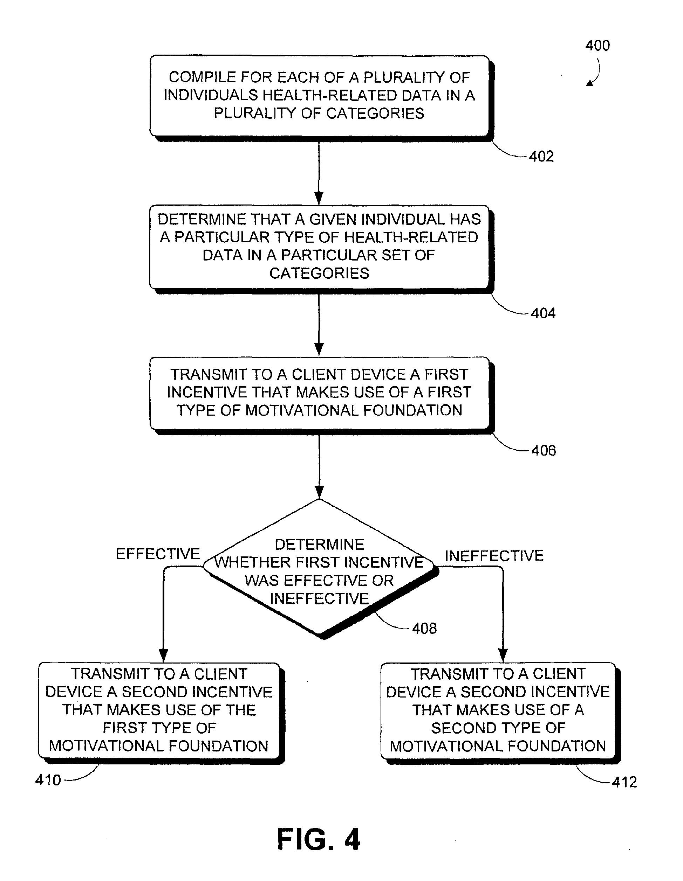

Figures 4 is a flowchart of an example method 400 that could be used as an incentive discovery process. The example method 400 may include one or more operations, functions, or actions, as depicted by one or more of blocks 402, 404, 406, 408, 410, and/or 412, each of which may be carried out by any of the systems described by way of Figures 1 and 2; however, other configurations could be used.

Furthermore, those skilled in the art will understand that the flowchart described herein illustrates functionality and operation of certain implementations of example embodiments. In this regard, each block of the flowchart may represent a module, a segment, or a portion of program code, which includes one or more instructions executable by a processor for implementing specific logical functions or steps in the process. The program code may be stored on any type of computer readable medium, for example, such as a storage device including a disk or hard drive. In addition, each block may represent circuitry that is wired to perform the specific logical functions in the process. Alternative implementations are included within the scope of the example embodiments of the present application in which functions may be executed out of order from that shown or discussed, including substantially concurrent or in reverse order, depending on the functionality involved, as would be understood by those reasonably skilled in the art.

Method 400 begins at block 402 at which the server-system compiles health-related data in a plurality of categories for each of a plurality of individuals. As described above, the server system may receive health-related data from any of a plurality of devices associated with an individual, such as client-devices including mobile telephones, personal or tablet computers, and wearable devices, and other data sources, such as those affiliated with an individual's health professional, or national or local organizations, such as the National Weather Service or the Centers for Disease Control. The server system may receive health-related data via any wired or wireless connection over one or more networks, including local area networks and wide area networks, such as the Internet. As also described above, the health-related data may be any data pertaining to an individual in any of a plurality of categories, including demographic data, environmental data, behavioral data, clinical data, and biomarker data, among other examples.

Continuing at block 404, the server system may determine that a given individual has a particular type of health-related data in a particular set of categories. For instance, in one example, the server system may determine that the individual's health-related data indicates that the individual (or someone associated with the individual, such as a health professional) has set a goal for the individual to lose 10 pounds within a year. Further, the server system may determine that the individual's health-related data currently indicates that the individual has not yet lost 10 pounds. In another example, the server system may determine that the individual's health-related data indicates that the individual has a BMI that is at an unhealthy level. Other examples of the server system making determinations that a given individual has a particular type of health-related data in a particular set of categories are possible as well.

Continuing at block 406, in response to determining that the given individual has a particular type of health-related data in a particular set of categories, the server system may transmit, over a communication network to a client device associated with the individual, a first incentive that makes use of a first type of motivational foundation. In order to transmit an incentive to a client device, the server system may, for instance, transmit an instruction to the client device that causes the client device to display or otherwise present the incentive. As described above, client devices associated with an individual may include any of a mobile telephone, a personal or tablet computer, and a wearable computing device, among other examples. As also described above, an incentive may generally take any form, including a message, alert, recommendation, or other communication presented at the client device. In some examples, an incentive may include a visual component, such as textual or graphical information displayed on a display, an auditory component (*e.g*., an alarm sound), and/or a tactile component *(e.g.,* a vibration), although other examples are possible.

The incentive may designed selected based on the individual's particular type of health-related data in the particular set of categories determined by the server system at block 404. As such, the incentive may be designed or selected to encourage or motivate the individual to engage in one or more behaviors to change the health-related data in the particular set of categories. Alternatively, the incentive may be designed or selected to encourage or motivate the individual to engage in one or more behaviors to change health-related data that may be in other categories as well. Consistent with the example described above, for instance, if the server system determines the individual's health-related data indicates that there is a goal for the individual to lose 10 pound within the year and that the individual has not yet lost 10 pounds, then the server system may design or select a first incentive that encourages or motivates the individual to exercise. Additionally or alternatively, the server system may design or select a first incentive that encourages or motivates the individual to alter the individual's diet. The server system may design or select any other incentive that encourages or motivates the individual to engage in any other behavior, including engaging in one or more actions or inactions, to change the individual's health-related data.

The first incentive may make use of a first type of motivational foundation. As described above, different incentives may make use of different types of motivational foundations, including by way of example, positive reinforcement, negative reinforcement, extrinsic motivations, and intrinsic motivations, among others. Consistent with the example described above, the server system may design or select an first incentive that makes use of, for instance, positive reinforcement and an intrinsic motivation. As an example, the server system may transmit an instruction that causes a client device to display an incentive that reads, "You have lost five pounds this year, and are half way to achieving your goal! Make sure to exercise today so that you can reach your goal!" Other examples of incentives are possible as well.

Continuing at block 408, the server system determines whether the first incentive was effective or ineffective. The server system may carry out this determination by referring back to the individual's health-related data to determine whether the individual engaged in the behavior for which the incentive was designed to encourage. In the example described above, the first incentive was designed to encourage the individual to exercise; thus, the server system may refer to the individual's health-related data to determine whether the individual actually exercised that day. If the health-related data indicates that the individual exercised that day, then the server system may conclude that the first incentive, which made use of positive reinforcement and an intrinsic motivation, was effective. In this case the flow may continue at block 410. However, if the health-related data indicates that the individual did not exercise that day, then the server system may conclude that the first incentive was ineffective.

As an alternative way to determine whether the first incentive was effective or ineffective, the server system may determine whether the individual's health-related data underwent a particular change, even though the individual may not have engaged in the particular behavior that the first incentive was designed to encourage. In the example described above, even if the individual's health-related data indicates that the individual did not exercise on the day the first incentive was sent, if the individual's health-related data eventually indicates that the individual met the goal of losing 10 pounds within a year, the server system may nonetheless consider the first incentive to be effective. In this case, flow may continue at block 410.

At block 410, the server system transmits, over a communication network to a client device associated with the individual, a second incentive that makes use of the first type of motivational foundation. Additionally, the server system may modify incentive-profile data of the individual to indicate that the first type of motivational foundation is effective for the individual, either on a general basis or on a behavior-specific basis. For instance, the server system may modify the incentive-profile data to indicate that the first type of motivational foundation is generally effective for all types of behaviors for the individual. Alternatively, the server system may modify the incentive-profile data to indicate that the first motivational foundation is effective for just those behaviors for which the server system determined that the first incentive was effective. Thus, in the example above, if the individual exercised on the day on which the first incentive encouraged the individual to exercise, then the server system may modify the incentive-profile data to indicate that the first motivational foundation is effective for motivating the individual to exercise. As the server system engages in additional incentive discovery processes for the individual, perhaps determining that the first motivational foundation is effective in motivating the individual to engage in other behaviors, the server system may modify the individual's incentive-profile data accordingly. In any case, when designing or selecting additional incentives for the individual, the server system may thereafter refer to the incentive-profile data and design or select incentives consistent with the types of motivational foundations indicated as being effective for that individual.

At block 412, after the server system determines that the first incentive, which made use of the first type of motivational foundation, was ineffective, the server system may transmit, over a communication network to a client device associated with the individual, a second incentive that makes use of a second type of motivational foundation. In the example described above, the first incentive was designed to encourage the individual to exercise and made use of positive reinforcement and an intrinsic motivation. Thus, for the second incentive, which may still be designed to encourage the individual to exercise, the server system may utilize negative reinforcement and an extrinsic motivation. For instance, the server system may select or design an incentive that reads, "70% of other women in your age group and location with similar occupations exercised today." Other examples are possible as well.

Additionally, the server system may modify incentive-profile data of the individual to indicate that the first type of motivational foundation is ineffective for the individual, either on a general basis or on a behavior-specific basis. For instance, the server system may modify the incentive-profile data to indicate that the first type of motivational foundation is generally ineffective for all types of behaviors for the individual. Alternatively, the server system may modify the incentive-profile data to indicate that the first motivational foundation is ineffective for just those behaviors for which the server system determined that the first incentive was ineffective. Thus, in the example above, if the individual failed to exercise on the day on which the first incentive encouraged the individual to exercise, then the server system may modify the incentive-profile data to indicate that the first motivational foundation is ineffective for motivating the individual to exercise. As the server system engages in additional incentive discovery processes for the individual, perhaps determining that the first motivational foundation is ineffective in motivating the individual to engage in other behaviors, the server system may modify the individual's incentive-profile data accordingly. In any case, when designing or selecting additional incentives for the individual, the server system may thereafter refer to the incentive-profile data and design or select incentives consistent with the types of motivational foundations indicated as being effective for that individual.

The server system may engage in one or more additional actions not depicted on flowchart 400. For example, after engaging in the incentive discovery process for several individuals and accordingly modifying respective incentive-profile data for each individual, the server system may analyze the incentive-profile data in order to identify patterns among individuals that share some health-related data. For instance, through the incentive discovery process and a pattern analysis, the server system may identify that at least a threshold percentage of individuals (*e.g.,* 75%) in a particular age group, with a particular occupation, and with similar exercise habits tend to motivated by the same type or types of motivational foundations. In response, the server system may provisionally modify incentive-profile data of additional individuals that have similar health-related data but for which the server system may not yet have engaged in an incentive discovery process. The server system may provisionally modify these additional individuals' incentive-profile data to indicate that the identified type or types of motivational foundations are effective for these additional individuals. As the server system engages in an incentive discovery process for these additional individuals, the server system may modify or update the individuals' incentive-profile data accordingly.

### IV. Conclusion

Where example embodiments involve information related to an individual or a device associated with an individual, the embodiments should be understood to include privacy controls. Such privacy controls include, at least, anonymization of device identifiers, transparency and user controls, including functionality that would enable users to modify or delete information relating to the user's use of a product. Further, in situations in where embodiments discussed herein collect personal information about individuals, or may make use of personal information, the individual may be provided with an opportunity to control whether programs or features collect information about the individual (*e.g.,* information about an individual's medical history, social network, social actions or activities, profession, an individual's preferences, or an individual's current location), or to control whether and/or how to receive content from the content server that may be more relevant to the individual.

The particular arrangements shown in the figures should not be viewed as limiting. It should be understood that other embodiments may include more or less of each element shown in a given figure. Further, some of the illustrated elements may be combined or even omitted. Yet further, an exemplary embodiment may include elements that are not illustrated in the figures.

Additionally, while various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims. Other embodiments may be utilized, and other changes may be made, without departing from the spirit or scope of the subject matter presented herein. It will be readily understood that the aspects of the present disclosure, as generally described herein, and illustrated in the figures, can be arranged, substituted, combined, separated, and designed in a wide variety of different configurations, all of which are contemplated herein.

## Claims

1. A method comprising: compiling, for each of a plurality of individuals, health-related data in a plurality of categories;
determining that a given individual in the plurality of individuals has a particular type of health-related data in a particular set of one or more categories;
based on the determination that the given individual has the particular type of health-related data in the particular set of one or more categories, transmitting from a server device to a client device associated with the given individual, over a communication network, a first instruction configured to cause the client device to present a first incentive designed to cause a change in the given individual's health-related data, wherein the first incentive makes use of a first type of motivational foundation; and
after transmitting the first instruction, determining whether the first incentive was effective or ineffective.

2. The method of claim 1, wherein the plurality of categories includes at least one of a demographic category, a behavioral category, a clinical-diagnosis category, an environmental category, and a biomarker-result category, and/or
wherein determining whether the first incentive was effective or ineffective comprises determining whether the given individual's health-related data underwent the change, and/or wherein determining whether the first incentive was effective or ineffective comprises determining whether the given individual engaged in one or more actions or inactions designed to cause the change in the given individual's health-related data, and/or
wherein the first incentive is designed to cause a change in the given individual's health-related data in at least one category of the particular set of one or more categories, and/or
further comprising:
in response to determining whether the first incentive was effective or ineffective,
selecting a second incentive designed to cause a change in the given individual's health-related data, such that the second incentive makes use of either the first type of motivational foundation or a type of motivational foundation different than the first type of motivational foundation based on the determination of whether the first incentive was effective or ineffective; and transmitting from the server device to the client device associated with the given individual, over the communication network, a second instruction configured to cause the client device to present the second incentive.

3. The method of claim 2, wherein the second incentive is designed to cause a change in the given individual's health-related data in at least one category of the particular set of one or more categories, and/or
wherein determining whether the first incentive was effective or ineffective comprises determining that the first incentive was effective, and wherein selecting a second incentive comprises selecting a second incentive that makes use of the first type of motivational foundation, and/or
wherein determining whether the first incentive was effective or ineffective comprises determining that the first incentive was ineffective, and/or
wherein selecting a second incentive comprises selecting a second incentive that makes use of a second type of motivational foundation, wherein the second type of motivational foundation is different than the first type of motivational foundation.

4. The method of claim 1, wherein the first type of motivational foundation comprises at least one of an extrinsic motivation, an intrinsic motivation, a positive reinforcement, or a negative reinforcement.

5. A system comprising:
one or more processors;
a communication interface; and
computer-readable storage media having stored thereon instructions that, when executed by the one or more processors, cause the system to engage in operations, the operations comprising:
compiling, for each of a plurality of individuals, health-related data in a plurality of categories;
determining that a given individual in the plurality of individuals has a particular type of health-related data in a particular set of one or more categories;
based on the determination that the given individual has the first type of health-related data in the particular set of one or more categories, transmitting from the system via the communication interface to a client device associated with the given individual a first instruction configured to cause the client device to present a first incentive designed to cause a change in the given individual's health-related data, wherein the first incentive makes use of a first type of motivational foundation; and
after transmitting the first instruction, determining whether the first incentive was effective or ineffective.

6. The system of claim 5, wherein the plurality of categories includes at least one of a demographic category, a behavioral category, a clinical-diagnosis category, an environmental category, and a biomarker-result category.

7. The system of claim 5, wherein determining whether the first incentive was effective or ineffective comprises determining whether the given individual's health-related data underwent the change.

8. The system of claim 5, wherein determining whether the first incentive was effective or ineffective comprises determining whether the given individual engaged in one or more actions or inactions designed to cause the change in the given individual's health-related data.

9. The system of claim 5, wherein the first incentive is designed to cause a change in the given individual's health-related data in at least one category of the particular set of one or more categories.

10. The system of claim 5, wherein the operations further comprise:
in response to determining whether the first incentive was effective or ineffective,
selecting a second incentive designed to cause a change in the given individual's health-related data, such that the second incentive makes use of either the first type of motivational foundation or a type of motivational foundation different than the first type of motivational foundation based on the determination of whether the first incentive was effective or ineffective; and
transmitting from the system via the communication interface to a client device a second instruction configured to cause the client device to present the second incentive.

11. The system of claim 10, wherein the second incentive is designed to cause a change in the given individual's health-related data in at least one category of the particular set of one or more categories.

12. The system of claim 10, wherein determining whether the first incentive was effective or ineffective comprises determining that the first incentive was effective, and wherein
selecting a second incentive comprises selecting a second incentive that makes use of the first type of motivational foundation.

13. The system of claim 10, wherein determining whether the first incentive was effective or ineffective comprises determining that the first incentive was ineffective, and
wherein selecting a second incentive comprises selecting a second incentive that makes use of a second type of motivational foundation, wherein the second type of motivational foundation is different than the first type of motivational foundation.

14. The system of claim 5, wherein the first type of motivational foundation comprises at least one of an extrinsic motivation, an intrinsic motivation, a positive reinforcement, or a negative reinforcement.
